(19) **Europäisches Patentamt**
**European Patent Office**
**Office européen des brevets**

(11) **EP 2 240 036 B1**

(12) **EUROPEAN PATENT SPECIFICATION**

(45) Date of publication and mention
of the grant of the patent:
**14.01.2015 Bulletin 2015/03**

(51) Int Cl.:
***A23L 1/303*** *(2006.01)*

(21) Application number: **09707867.9**

(86) International application number:
**PCT/EP2009/051384**

(22) Date of filing: **06.02.2009**

(87) International publication number:
**WO 2009/098295 (13.08.2009 Gazette 2009/33)**

(54) **PULVERULENT COMPOSITION AND A PROCESS FOR PREPARING THE SAME**

PULVERFÖRMIGE ZUSAMMENSETZUNG UND VERFAHREN ZU IHRER HERSTELLUNG

COMPOSITION PULVÉRULENTE ET SON PROCÉDÉ DE PRÉPARATION

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **06.02.2008 EP 08290105**

(43) Date of publication of application:
**20.10.2010 Bulletin 2010/42**

(73) Proprietor: **Innov'ia**
**17000 La Rochelle (FR)**

(72) Inventor: **GRIZEAU, Alain**
**F-17000 La Rochelle (FR)**

(74) Representative: **Grosset-Fournier, Chantal
Catherine et al
Grosset-Fournier & Demachy
54, rue Saint-Lazare
75009 Paris (FR)**

(56) References cited:
**EP-A- 0 116 755        EP-A- 0 413 828
EP-A- 1 198 994        EP-A2- 1 249 180
WO-A-00/64277        DE-A1- 1 492 034
GB-A- 892 914        JP-A- 2003 267 867**

Remarks:
The file contains technical information submitted after
the application was filed and not included in this
specification

**EP 2 240 036 B1**

**Description**

The present invention relates to a pulverulent composition and to a process for preparing the same.

More particularly, the invention relates to stable pulverulent compositions having instantaneous dispersion and solubility properties in aqueous solutions, said pulverulent compositions containing substances, in particular vitamins, which are poorly soluble in aqueous media.

Some vitamins, such as vitamin E, vitamin D (D2 Ergocalciferol, D3 cholecalciferol), vitamin A and pro vitamin A, vitamin B8 (Biotin), vitamin B9 (Folic Acid), vitamin B12 (cyanocobalamin and other cobalamin) and vitamin B2 (Riboflavin) (French Patent FR 1 211 662), do not disperse well in aqueous solution. When those vitamins are added to food products or drinks to prepare vitamin supplemented food product or drinks, some lumps or spots are observed on the surface of the food product or on the wall of the container containing the drink, which is not pleasant for the consumer.

One of the aspects of the present invention is to provide a pulverulent composition having instantaneous dispersion and solubility properties in aqueous solutions.

One of the aspects of the present invention is to provide a process of preparation of a pulverulent composition having instantaneous dispersion and solubility properties in aqueous solutions.

One of the aspects of the present invention is to provide foodstuff and drinks containing a pulverulent composition having instantaneous dispersion and solubility properties in aqueous solutions.

The present invention relates to a pulverulent composition comprising less than 96% in weight, preferably from 10 to 80%, and more preferably from 20 to 70%, in particular from 30 to 60%, particularly 44 to 55%, of at least a vitamin having a solubility lower than 20g/L, particularly lower than 10g/L, in particular lower than 5g/L, and preferably lower than 1g/L and more preferably lower than 500mg/L in aqueous media, said pulverulent composition being stable on storage and having instantaneous dispersion and solubility properties in aqueous solutions.

The present invention relates to a pulverulent composition comprising more than 15% in weight, and less than 96% in weight, preferably from 15 to 80%, and more preferably from 20 to 70%, in particular from 30 to 60%, particularly 44 to 55%, of at least a vitamin having a solubility lower than 20g/L, particularly lower than 10g/L, in particular lower than 5g/L, and preferably lower than 1g/L and more preferably lower than 500mg/L in aqueous media, said pulverulent composition consisting in particles individually coated by at least one hydrophilic agent, and said pulverulent composition being stable on storage and having instantaneous dispersion and solubility properties in aqueous solutions.

By "pulverulent composition" is meant a composition of one or several substances in powder form.

By "vitamin" is meant a nutrient that is an organic compound required in tiny amounts for essential metabolic reactions in a living organism.

By "aqueous medium" is meant a medium in which the major component is water, in particular an aqueous solution.

By "stable on storage" is meant that the pulverulent composition is stable when stored in the original unopened packaging in a dry room (with a hygroscopicity lower than 65% and a temperature comprised between 10 and 25°C) for one year.

By "instantaneous dispersion in aqueous solutions" is meant that when the pulverulent composition is poured into the aqueous solutions, it disperses entirely and instantaneously. For instance, for 10g of powder added to 100ml distilled water at room temperature, the dispersibility is characterised by the time needed for the powder to be wet by the water and disperse into the water, not standing at the surface of the water. If the time needed for wettability by water and dispersion is shorter than 15 seconds the dispersibility is considered as instant.

By "instantaneous solubility properties in aqueous solutions" is meant that the pulverulent composition dissolves entirely in the aqueous medium to make a solution. For instance, for 10g of powder added to 100ml distilled water at room temperature, the solubility is characterised by the quantity of lumps or insoluble particles in the water 10 mn after adding the powder in the water. No lumps at the surface and no insoluble particles at the bottom of the recipient.

The invention relates to a pulverulent composition as described above, wherein the vitamin is chosen among the group consisting of vitamin E, vitamin B2 (Riboflavin), vitamin D (D2 Ergocalciferol, D3 cholecalciferol), vitamin A and pro vitamin A, vitamin B8 (Biotin), vitamin B9 (Folic Acid), and vitamin B12 (cyanocobalamin and other cobalamin), preferably wherein the vitamin is riboflavin.

Said vitamins present a low solubility in aqueous medium.

Riboflavin has a solubility of 70mg/L water.

The invention relates to a pulverulent composition as described above, wherein the vitamin is chosen among the group consisting of vitamin E, vitamin B2 (Riboflavin), vitamin A and pro vitamin A, vitamin B8 (Biotin), vitamin B9 (Folic Acid), and vitamin B12 (cyanocobalamin and other cobalamin), preferably wherein the vitamin is riboflavin

The invention relates to a pulverulent composition as described above, comprising at least one saccharide, chosen among the group consisting of monosaccharide, disaccharide and polysaccharide.

The invention relates to a pulverulent composition as described above, wherein the hydrophilic agent comprises or is constituted by at least one saccharide, in particular chosen among the group consisting of monosaccharides, disaccharides and polysaccharides.

By "saccharide" is meant sugar, also called carbohydrates. Monosaccharides consist of one sugar. Examples of monosaccharides include glucose (dextrose), fructose, galactose, xylose and ribose. Monosaccharides are the building blocks of:

- disaccharides (consisting of two sugar) like sucrose (common sugar) and,
- polysaccharides (such as cellulose and starch). Polysaccharides are polymers made up of many monosaccharides joined together by glycosidic bonds.

The present invention relates to a pulverulent composition as described above, wherein the hydrophilic agent comprises or consists in:

- at least a first saccharide possessing hydrocolloidal properties, said saccharide being preferably chosen among the group consisting of cellulose, alginate, starch, modified starch, in particular octenyl succinate starch, arabic gum, and,
- at least a second saccharide, chosen among the group consisting of
- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen and starch or modified starch, fructooligosaccharide (FOS).

The expression *"hydrocolloidal properties"* means forming colloidal dispersion, and/ or forming colloidal gel when dispersed in water.

The first saccharide makes a matrix around the substance or vitamin thanks to its hydrocolloidal properties, and the second saccharide participates to the matrix of the first saccharide to complete the good properties of the pulverulent composition with respect to its behaviour in water.

The present invention relates to a pulverulent composition as described above, wherein the pulverulent composition comprises from 5% to 95%, preferably 20% to 70%, in particular 30 to 60%, and more preferably from 40% to 60% in weight of saccharide.

The present invention relates to a pulverulent composition as described above, wherein the pulverulent composition comprises from 5% to 85%, preferably 20% to 70%, in particular 30 to 60%, and more preferably from 40% to 60% in weight of saccharide.

The present invention relates to a pulverulent composition as described above, wherein the vitamin is riboflavin, the first saccharide is octenyl succinate starch and the second saccharide is glucose syrup.

By "starch" is meant a mixture of amylose and amylopectin (chemical formula $(C_6H_{10}O_5)_n$). These are both complex carbohydrate polymers of glucose.

By "octenyl succinate starch" is meant octenyl succinate starch with a degree of substitution comprised between 0.01 and 0.10, preferably between 0.01 and 0.04, and more preferably around 0.02.

Octenyl succinate starch is used for its specific emulsifying properties allowing good dispersion of the matrix around each particle of substance or vitamin. Glucose sirup with its smaller glucose polymer chains complete the matrix between the spaces left by the longer chains of starch and participate to the water absorption when the pulverulent composition is rehydrated.

The present invention relates to a pulverulent composition as described above, wherein the mean diameter of said particle is from 0.2 $\mu$m to 50 $\mu$m, preferably 0.5 $\mu$m to 30 $\mu$m, more preferably 1 $\mu$m to 20 $\mu$m.

The present invention relates to a pulverulent composition as described above, characterized in that the pulverulent composition comprises granules, said granules consisting in a set of particles, and wherein:

- said granules comprise at least a vitamin having a solubility lower than 20g/L and preferably 1g/L and more preferably lower than 500mg/L in aqueous media and at least a saccharide,
- the mean diameter of said granule is from 10 $\mu$m to 500 $\mu$m, preferably 20 to 100 $\mu$m, more preferably 30 to 50 $\mu$m,
- the span (DV05) of said granules is from 0.2 to 3.0, preferably 1.0 to 2.0, more preferably from 1.2 to 1.7,
- the macroscopic density of said pulverulent composition is from 300 to 800 g/l, preferably 400 to 700 g/l, and more preferably 400 to 600 g/l,
- the flow index determined by Flowdex method of said pulverulent composition is from 4 to 20, preferably from 5 to 10, more preferably around 8.

It is relevant to make a distinction between the terms particles and granules.

A particle is a solid constituted by one single substance. The particle shape is defined by its origin, its chemical composition and its manufacturing process (e. g. a crystal obtained by crystallization, a dried drop obtained by a drying process, individual fiber obtained through grinding).

The granule is the macroscopic form of the pulverulent composition.

The granules are constituted by a set of particles. Said particles may be identical or different from each other.

Span is a dispersion parameter obtained in granulometer measurement by laser diffraction using three median parameters of the particle distribution curve criteria (90% Passing diameter D(v,0.90), 50% passing diameter D(v,0.50) and 10% passing diameter D(v,0.10)) as follows:

$$\text{Span} = [D(v,0.10) - D(v,0.90)] / D(v,0.50).$$

The "Flowdex method" measures the flowability of a powder measuring the smallest hole diameter of a stainless steel disc where the powder can flow, which is the Flowdex index. Flowdex device is composed of a cylinder with the interchangeable discs with holes of various diameters at the bottom. The determination of fluidity is based on the capacity of the powder to fall freely by a hole in the disc. The powder is carefully charged on the top of the hole. If the hole through which a powder falls freely is small, the flowability is good, when the hole is larger, the worse is the flowability.

The method FLOWDEX initially developed and discussed by Gioia A. Intrinsic flowability: a new technology for powder flowability classification, Pharmaceut. Technol. (1980) 1-4

The pulverulent composition as described above is the one obtained after the atomisation process as detailed hereafter.

The present invention relates to a pulverulent composition as described above, wherein said granules comprise riboflavin, modified starch, in particular octenyl succinate starch and glucose syrup.

The present invention relates to a pulverulent composition as described above, characterized in that the pulverulent composition comprises granules, wherein:

- said granules comprise at least a vitamin having a solubility lower than 20g/L and preferably 1g/L and more preferably lower than 500mg/L in aqueous media and at least a saccharide,
- the mean diameter of said granule is from 10 $\mu$m to 500 $\mu$m, preferably 20 to 100 $\mu$m, more preferably 30 to 50 $\mu$m,
- the span (DV05) of said granules is from 0.2 to 3.0, preferably 1.0 to 2.0, more preferably from 1.2 to 1.7,
- the macroscopic density of said pulverulent composition is from 300 to 800 g/l, preferably 400 to 700 g/l, and more preferably 400 to 600 g/l,
- the flow index determined by Flowdex method of said pulverulent composition is from 4 to 20, preferably from 5 to 10, more preferably around 8,
- the stability of a solution made of 1,5 g of said pulverulent composition in 8,5 g of water is such that a sediment from 1,5 ml to 10 ml, preferably 4 to 9 ml , more preferably from 6 to 8 ml is obtained after 24 hours.

By "sediment" is meant any particulate matter that can be transported by fluid flow and which eventually is deposited as a layer of solid particles on the bed or bottom of a body of the solution.

By "obtained after 24 hours" is meant 24 hours after the pulverulent composition was poured into water.

The pulverulent composition as described above is the one obtained after the microgrinding and atomisation process as detailed hereafter.

The present invention relates to a pulverulent composition as described above, wherein said granules comprise riboflavin, modified starch, in particular octenyl succinate starch and glucose.

The pulverulent composition is composed of granules, which are the unit element of the pulverulent composition.

The present invention also relates to a granule comprising at least a vitamin having a solubility lower than 20g/L and preferably 1g/L and more preferably lower than 500mg/L in aqueous media, and at least a saccharide wherein:

- the mean diameter of said granule is from 10 $\mu$m to 500 $\mu$m, preferably 20 to 100 $\mu$m, more preferably 30 to 50 $\mu$m,
- the macroscopic density of said granule is from 300 to 800 g/l, preferably 400 to 700 g/l, and more preferably 400 to 600 g/l,
- the time to disperse and solubilize 10 grams of said granules in 100 mL of an aqueous solutions is from 2 min to 5 s, preferably 1 min to 5s, and more preferably less than 1 min, said granule comprising preferably riboflavin, glucose and modified starch, in particular octenyl succinate starch.

In another embodiment, the present invention also relates to a process of preparation of pulverulent compositions as described above and of granules constituting said pulverulent compositions as described above comprising:

1) possibly a step of mixing a vitamin having a solubility lower than 20g/L and preferably 1g/L and more preferably lower than 500mg/L in aqueous media with at least a saccharide to obtain an aqueous phase comprising a vitamin and at least one saccharide, and,

2) possibly a step of microgrinding of the aqueous phase comprising a vitamin and at least one saccharide to obtain a microgrinded aqueous phase comprising a vitamin and at least one saccharide, and,

3) a step of atomisation of the microgrinded aqueous phase comprising a vitamin and at least one saccharide to obtain a mixture comprising a vitamin and at least one saccharide, said mixture being constituted by granules;

the (possibly microgrinded) aqueous phase comprising a vitamin and at least one saccharide is poured into an atomisation tower and is transformed into a mixture; said mixture is a solid and appears like a dust, constituted of granules; said mixture is either the final product, i.e. the pulverulent composition according to the invention, or an intermediary when a step of coating of the granules is added to the process (i.e. step 4));

4) possibly a step of coating of said granules to obtain a pulverulent composition, and,

5) possibly a step of recovery of the pulverulent composition obtained.

In another embodiment, the present invention also relates to a process of preparation of pulverulent compositions and of granules constituting said pulverulent compositions as described above comprising a step of atomisation of an aqueous phase comprising a vitamin and at least one saccharide, said vitamin having a solubility lower than 20g/L and preferably 1g/L and more preferably lower than 500mg/L in aqueous media.

By "Atomisation", also called "spray drying" is meant conversion of the aqueous phase comprising a vitamin and of at least one saccharide into a spray or mist (i.e. collection of drops), by passing the aqueous phase through a nozzle.

When appropriate ingredients are used in the aqueous phase, in particular saccharide, this process allows the preparation of a pulverulent composition having instantaneous dispersion and solubility properties in aqueous solutions.

The present invention also relates to a process of preparation of pulverulent compositions as described above and of granules constituting said pulverulent compositions as described above comprising:

- a step of mixing a vitamin having a solubility lower than 20g/L and preferably 1g/L and more preferably lower than 500mg/L in an aqueous media with at least a saccharide to obtain an aqueous phase comprising a vitamin and at least one saccharide, and,
- a step of atomisation of said aqueous phase comprising a vitamin and at least one saccharide to obtain a mixture comprising a vitamin and at least one saccharide, said mixture being constituted by granules, and,
- possibly a step of coating of said granules to obtain a pulverulent composition.

By "coating" is meant a covering that is applied to the granule to protect it or change its appearance. Such coating can be hydrocolloids as cellulose ingredients allowing protection against off flavors, or gums allowing gastro-resistant properties of the pulverulent composition as examples.

The present invention also relates to a process as described above in which a step of microgrinding of the aqueous phase comprising a vitamin and at least one saccharide is added prior to the step of atomisation.

By "microgrinding" is meant reducing the size of the particle to micronic size (less than $50\mu m$ and preferably less than $10\mu m$).

The present invention also relates to a process of preparation of pulverulent compositions as described above and of granules constituting said pulverulent compositions as described above comprising:

- a step of mixing a vitamin having a solubility lower than 20g/L and preferably 1g/L and more preferably lower than 500mg/L in aqueous media with at least a saccharide to obtain an aqueous phase comprising a vitamin and at least one saccharide, and,
- possibly a step of microgrinding of the aqueous phase comprising a vitamin and at least one saccharide to obtain a microgrinded aqueous phase comprising a vitamin and at least one saccharide, and,
- a step of atomisation of the microgrinded aqueous phase comprising a vitamin and at least one saccharide to obtain a mixture comprising a vitamin and at least one saccharide, said mixture being constituted by granules, and,
- possibly a step of coating of said granules to obtain a pulverulent composition, and,
- a step of recovery of the pulverulent composition obtained.

In this embodiment, the pulverulent composition is recovered at the end of the process.

In another embodiment, the present invention relates to the use of a pulverulent composition as described above or/and of granules constituting said pulverulent compositions as described above to prepare:

- liquid food products such as infantile milk, milk, acidified milk products, drinks, fruit juices and sorbet, or,
- solid food products such as bakery, noodles, bread, or,

- semi-liquid food products such as spreads, yoghourts, dips and ice creams, or,
- emulsion food products such as sauces, in particular mayonnaise and mustard,-
- powders such as dehydrated soup, dehydrated sauce, dehydrated drink preparation, powder milk, cocoa powder, instant powder drinks, meal replacement products or effervescent products, said powders being liable to be instantaneously dispersed in an aqueous solution to provide a beverage,
- a pharmaceutical composition.

The advantage of using the pulverulent composition or/and the granules according to the invention in the preparation of liquid food products, solid food products, semi-liquid food products, emulsion food products, powders, and of a pharmaceutical composition is that the appearance of these products/powders/composition will be improved in comparison with products/powders/composition for the preparation of which a vitamin having a solubility lower than 20g/L and preferably 1g/L and more preferably lower than 500mg/L in aqueous media is used. The colour will be more homogeneous. Fewer or no lighter or darker spots or dots will be observed on the surface or inside the products/powders/composition, which is more pleasant for the consumer.

The pharmaceutical composition contains excipient and an active substance, which is either the vitamin having a solubility lower than 20g/L and preferably 1g/L and more preferably lower than 500mg/L in aqueous media, or another component of the pulverulent composition according to the invention.

In another embodiment, the present invention relates to a liquid food product containing:

- from 0,1 mg/kg to 1500 mg/kg, preferably 1 mg/kg to 400 mg/kg, more preferably 4 mg/kg to 40 mg/kg of a pulverulent composition as described above,
- from 0,1 mg/kg to 1500 mg/kg, preferably 1 mg/kg to 400 mg/kg, more preferably 4 mg/kg to 40 mg/kg of granules constituting said pulverulent compositions as described above, wherein:

    - said liquid food product is liquid or frozen,
    - said liquid food product is stable at temperatures from 6 to 60°C, preferably from 10°C to 50°C, more preferably from 20°C to 40°C during a period of 2 years,
    - said pulverulent composition or granule is homogeneously dispersed in said liquid food product,
    - said vitamin does not form any deposit on the wall of a container which would contain said liquid food product,
    - said liquid food product being preferably chosen among the group consisting of oil, infantile milk, milk, acidified milk products, drinks, fruit juices and sorbet,
      except liquid food product chosen from the group consisting of infantile milk, milk, acidified milk products, drinks and oils and containing :
    - either a pulverulent composition,
    - or granules constituting said pulverulent composition,
      said pulverulent composition or said granules comprising:
    - riboflavin,
    - a first saccharide possessing hydrocolloidal properties, and/or
    - a second saccharide, chosen among the group consisting of

        - monosaccharide such as fructose, glucose, galactose,
        - disaccharide such as saccharose, sucrose, lactose, maltose, trehalose and cellobiose, and,
        - polysaccharide such as glucose syrup, dextrin, inulin, cellulose, glycogen, starch and modified starch, fructooligosaccharide (FOS).

In another embodiment, the present invention also relates to a solid food product containing:

- from 1 mg/kg to 1000 g/kg, preferably 10 mg/kg to 500 g/kg, more preferably 0,1 g/kg to 100 g/kg of a pulverulent composition as described above, or,
- from 1 mg/kg to 1000 g/kg, preferably 10 mg/kg to 500 g/kg, more preferably 0,1 g/kg to 100 g/kg of granules constituting said pulverulent compositions as described above,
  wherein:

    - said solid food product is stable at temperatures from 6 to 60°C, preferably from 10°C to 50°C, more preferably from 20°C to 40°C during a period of 2 years,
    - said pulverulent composition or granule is homogeneously dispersed in said solid food product,
    - said solid food product being preferably chosen among the group consisting of confectionery, bakery, noodles, bread, rice, cereals,

except solid food product containing :

- either a pulverulent composition,
- or granules constituting said pulverulent composition,

said pulverulent composition or said granules comprising:

- riboflavin,
- a first saccharide possessing hydrocolloidal properties, and/or
- a second saccharide, chosen among the group consisting of

  - monosaccharide such as fructose, glucose, galactose,
  - disaccharide such as saccharose, sucrose, lactose, maltose, trehalose and cellobiose, and,
  - polysaccharide such as glucose syrup, dextrin, inulin, cellulose, glycogen, starch and modified starch, fructooligosacchride (FOS).

In another embodiment, the present invention also relates to a semi-liquid food product containing:

- from 0,1 mg/kg to 1500 mg/kg, preferably 1 mg/kg to 400 mg/kg, more preferably 4 mg/kg to 40 mg/kg of a pulverulent composition as described above,
- from 0,1 mg/kg to 1500 mg/kg, preferably 1 mg/kg to 400 mg/kg, more preferably 4 mg/kg to 40 mg/kg of granules constituting said pulverulent compositions as described above, wherein:

  - said semi-liquid food product is semi-liquid or frozen,
  - said semi-liquid food product is stable at temperatures from 6 to 60°C, preferably from 10°C to 50°C, more preferably from 20°C to 40°C during a period of 2 years,
  - said pulverulent composition or granule is homogeneously dispersed in said semi-liquid food product,
  - said semi-liquid food product being preferably chosen among the group consisting of spreads, jams, yoghourts, dips and ice creams,
  - except semi-liquid food product chosen from the group consisting of spreads and jams and containing :
  - either a pulverulent composition,
  - or granules constituting said pulverulent composition,
    said pulverulent composition or said granules comprising:
  - riboflavin,
  - a first saccharide possessing hydrocolloidal properties, and/or
  - a second saccharide, chosen among the group consisting of

    - monosaccharide such as fructose, glucose, galactose,
    - disaccharide such as saccharose, sucrose, lactose, maltose, trehalose and cellobiose, and,
    - polysaccharide such as glucose syrup, dextrin, inulin, cellulose, glycogen, starch and modified starch, fructooligosaccharide (FOS).

In another embodiment, the present invention also relates to an emulsion food product containing:

- from 0,1 mg/kg to 1500 mg/kg, preferably 1 mg/kg to 400 mg/kg, more preferably 4 mg/kg to 40 mg/kg of a pulverulent composition as described above,
- from 0,1 mg/kg to 1500 mg/kg, preferably 1 mg/kg to 400 mg/kg, more preferably 4 mg/kg to 40 mg/kg of granules constituting said pulverulent compositions as described above, wherein:

  - said emulsion food product is liquid or frozen,
  - said emulsion food product is stable at temperatures from 6 to 60°C, preferably from 10°C to 50°C, more preferably from 20°C to 40°C during a period of 2 years,
  - said pulverulent composition or granule is homogeneously dispersed in said emulsion food product,
  - said emulsion food product is a sauce, in particular mayonnaise and mustard.

In another embodiment, the present invention also relates to a powder containing:

  * - from 1 mg/kg to 999 g/kg, preferably 10 mg/kg to 500 g/kg, more preferably 0,1 g/kg to 100 g/kg of a pulverulent composition as described above, or,

- from 1mg/kg to 999 g/kg, preferably 10 mg/kg to 500 mg/kg, more preferably 0,1 g/kg to 100 g/kg of granules constituting said pulverulent compositions as described above, and,

* at least a foodstuff being preferably chosen among the group consisting of dehydrated soup, dehydrated sauce, dehydrated drink preparation, powder milk, cocoa powder, instant powder drinks, meal replacement products and effervescent products,
wherein:

- said powder is stable at temperatures from 6 to 60°C, preferably from 10°C to 50°C, more preferably from 20°C to 40°C during a period of 2 years,
- said pulverulent composition or granule is homogeneously dispersed in said powder,
- said powder is liable to be instantaneously dispersed in an aqueous solution to provide a beverage,

except powders containing:

- either a pulverulent composition or granules constituting said pulverulent composition, said pulverulent composition or said granules comprising:

  - riboflavin,
  - a first saccharide possessing hydrocolloidal properties, and/or
  - a second saccharide, chosen among the group consisting of

    - monosaccharide such as fructose, glucose, galactose,
    - disaccharide such as saccharose, sucrose, lactose, maltose, trehalose and cellobiose, and,
    - polysaccharide such as glucose syrup, dextrin, inulin, cellulose, glycogen, starch and modified starch, fructooligosaccharide (FOS), and,

- a foodstuff being chosen among the group consisting of powder milk, cocoa powder, instant powder drinks, meal replacement products and effervescent products.

The present invention also relates to a powder as described above, wherein said foodstuff is soluble in an aqueous solution and preferably chosen among the group consisting of dehydrated drink preparation, instant powder drinks and effervescent products, and wherein said powder is liable to be dissolved in an aqueous solution to provide a beverage.

In this embodiment, as the foodstuff and the pulverulent composition and/or the granules are soluble in the aqueous solution, the beverage reconstituted by mixing the powder with an aqueous solution is a homogeneous solution just after said mixing is done. No suspension, spot, dot or emulsion is observed in the beverage.

In another embodiment, the present invention also relates to a process of preparation of a liquid food product as described above, comprising a step of instantaneously dispersing a powder as described above in an aqueous solution.

In this embodiment, the beverage which is obtained by mixing the powder according to the invention with an aqueous solution, is the liquid food product according to the invention.

The present invention also relates to a process as described above, comprising an additional phase of dissolving a powder as described above in an aqueous solution.

In this embodiment, the beverage, which is obtained by mixing the powder according to the invention with an aqueous solution and is a homogeneous solution, is the liquid food product according to the invention.

In another embodiment, the present invention also relates to a pharmaceutical composition containing:

- an active substance contained in the pulverulent composition as described above, or,
- an active substance contained in granules as described above,

in association with a pharmaceutically acceptable vehicle.

The pulverulent composition according to the invention contains the active principle of the pharmaceutical composition. In an embodiment, said active principle is the vitamin having a solubility lower than 20 g/L and preferably 1 g/L and more preferably lower than 500mg/L in aqueous media. In another embodiment, said active principle is another compound comprised in the pulverulent composition according to the invention, and the vitamin is one of the excipient.

In another embodiment, the present invention also relates to a pharmaceutical composition as described above, wherein the pulverulent composition or the granules comprise(s) riboflavin.

In an embodiment, the active principle is riboflavin. In another embodiment, the active principle is another compound comprised in the pulverulent composition according to the invention, and riboflavin is one of the excipient.

## FIGURES

**Figure 1:**

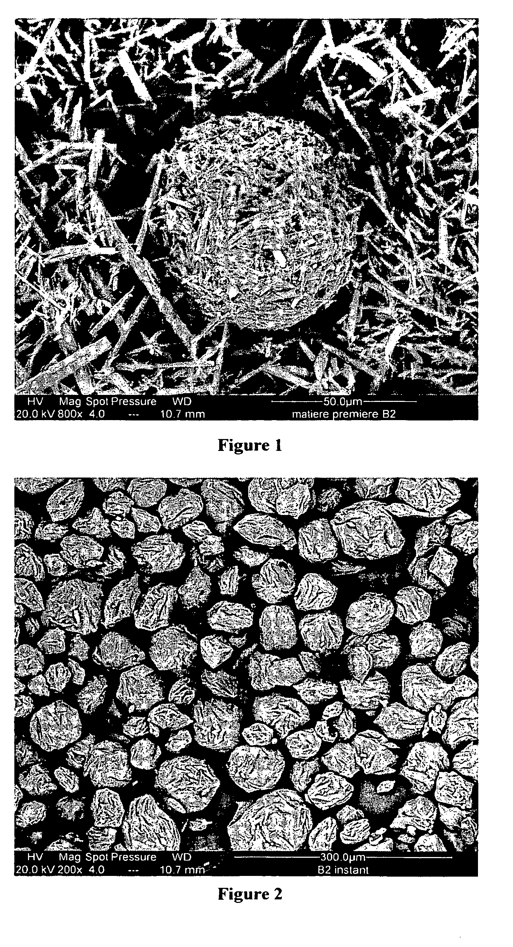

Figure 1 represents pure riboflavin observed on Electronic Scanning Microscopy.

**Figure 2:**
Figure 2 represents the pulverulent composition A according to the invention observed on Electronic Scanning Microscopy.

**Figure 3:**

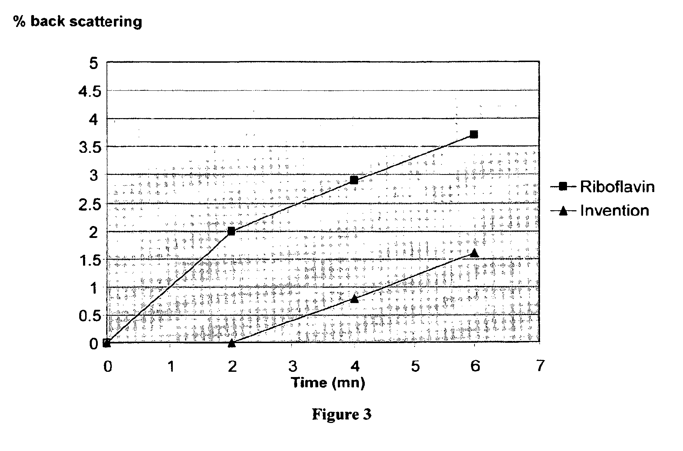

Figure 3 represents the sedimentation measured by turbidity using a back scattering laser device (Turbiscan MA 1000) of a dispersion of the pulverulent composition A according to the invention compared to the one of pure riboflavin. The percentage of back scattering is represented as a function of time.

## EXAMPLES

**Example 1 : Ingredients of the pulverulent compositions A and B.**

The riboflavin water-dispersible pulverulent compositions A and B have been made in accordance with the invention and contain the ingredients disclosed in Table 1.

*Table 1: Ingredients of the pulverulent compositions A and B.*

| Ingredient | % by total weight | Raw material reference |
|---|---|---|
| Riboflavin | 43.00 | Riboflavin (Hubei Guangji Pharmaceutical) |
| OSA | 14.25 | Sodium Octenyl Succinate starch (Cerestar) |
| Glucose syrup | 42.75 | Glucose syrup DE 38 (Cerestar) |
| % is based on dry matter of each component | | |

Pulverulent composition A was obtained after spray drying and pulverulent composition B was obtained after microgrinding followed by spray drying.

**Example 2: Detailed protocol for manufacture of water-dispersible pulverulent composition A obtained by spray drying.**

The ingredients of the composition are the ones disclosed in example 1.

0.76 kg of sodium octenyl succinate (OSA) and 2.26 kg of glucose syrup are added to 4.82 kg of water and complete dissolution is achieved. 2.16 kg of riboflavin are added and stirred vigorously to obtain a uniform suspension. The above suspension is then spray dried at a rate of 11 kg per hour. The inlet air (air coming in the atomisation tower) temperature is held at about 150°C and the outlet (air coming out of the atomisation tower) temperature is about 100°C. The pulverulent composition A obtained disperses instantaneously in water and results in a stable suspension before uses.

Granules of the pulverulent composition A obtained with the invention have been observed on Electronic Scanning Microscopy and the encapsulation can be observed surrounding the insoluble vitamin. The granules according to the invention obtained have a shape which is different from the one of pure vitamin (Figure 1 and 2).

**Example 3 : Protocol for testing the water-dispersible pulverulent composition A of example 2.**

The stability of the solution of the pulverulent composition A obtained in example 2 in water can be determined by the following laboratory tests.

**A- Sedimentation of dispersed pulverulent composition A in water measured by turbidity.**

10 g of the water-dispersible pulverulent composition A is dispersed in 90 ml of distilled water at 20°C in a tube. The sedimentation rate is measured by turbidity using a back scattering laser device (Turbiscan MA 1000) along the tube. The back light is measured along the tube at different time so that evolution of the sedimentation can be followed along time. The sedimentation is measured at the bottom of the tube. The following results show that the pulverulent composition A sediments at a lower rate compared to the control, which is the sedimentation of pure riboflavin (Table 2 and figure 3).

*Table 2: % Back light after 2 and 6 minutes according to the nature of the composition.*

| composition | % Back light after 2 min dispersion | % Back light after 6 min dispersion |
|---|---|---|
| 100% Riboflavin | 2% | 3.1% |
| Water-dispersible pulverulent composition A of example 2 | 0% | 1.6% |

**B- Sedimentation of dispersed pulverulent composition A in water measured by natural sedimentation in tube.**

The water-dispersible pulverulent composition A is dispersed in distilled water at 20°C at a concentration of 15% (net w/ total w) in a conical graduated sedimentation tube (total volume of the solution is 10ml). The quantity of the sediment (in ml) is measured along time (Table 3).

The encapsulation efficiency can also be assessed by measuring the hydration of the granules of the water-dispersible pulverulent composition of the invention. This has been evaluated by measuring the dry solid content of the sediment (% dry matter) (Table 3).

*Table 3: Sediment volume and percentage of dry solids content of the sediments depending on the nature of the composition.*

| composition | Sediment volume after 1 Hour dispersion (ml) | Dry solids content of the sediment after 24Hour sedimentation (%) |
|---|---|---|
| 100% Riboflavin (control) | 0.6 | 39.3% |
| Water-dispersible pulverulent composition A | 0.1 | 19.3% |

The sedimentation occurs more slowly with the pulverulent composition A of the invention compared to the control. The hydration of the sediment shows that the pulverulent composition A of the invention is more hydrated than the control and consequently better dispersed in the aqueous solution.

**Example 4: Detailed protocol for manufacture of water-dispersible pulverulent composition B obtained by grinding followed by spray drying.**

The ingredients of the composition are those disclosed in example 1.

0.76 kg of sodium octenyl succinate (OSA) and 2.26 kg of glucose syrup are added to 4.82 kg of water and complete dissolution is achieved. 2.16 kg of riboflavin are added and stirred vigorously to obtain a uniform suspension. The suspension is then grinded in a ball mixer (Wab laboratory device - Ball size 0.7-0.9mm). The above suspension is then spray dried. The inlet air temperature is held at about 150°C and the outlet temperature is about 100°C. The pulverulent composition B obtained disperses instantaneously in water and results in a stable suspension before uses.

**Example 5 : Stability of the pulverulent compositions A and B obtained in example 2 and 4**

The stability is measured by the sedimentation test as follow:

- 10 ml of solution is prepared using 8.5 ml of demineralised water and 1.5 g of the pulverulent composition A or B in special sedimentation tubes graduated from 0.1 ml up to 10 ml;
- complete dispersion is assumed by a 2 min vortex mixing;

- sedimentation is controlled at defined times to evaluate the amount of sediment;
- quality of the dispersion is evaluated by measuring :

    o the dispersion of the solids all over the aqueous solution by measuring the height of the sediments of the hydrated particles. For the same amount of pulverulent composition, the higher the sediments, the more dispersed the particles in the aqueous solution
    o the dry content of the sediment after 24 hours.

Results are described in table 4:

*Table 4: Size of the sediment and percentage of dry content of the sediment according to the nature of the composition.*

| Composition | | Sediment after 24H (ml) | Dry content of the sediment (%) |
|---|---|---|---|
| Riboflavine: Raw material (control) | | 1,5 | 39,4 |
| Composition of the invention A | spray dried | 2,4 | 28,3 |
| Composition of the invention B | grinded and spray dried | 7,0 | 6,6 |

Results show that the sediment of the composition A is more hydrated than the raw material (28 % dry content compared to approx 39% dry content for the raw material). The comparison of the results for pulverulent composition A and B also demonstrates the effect of pre-grinding of the material by the important increase in moisture (only 6.6% solids) and size of the sediment (7 ml), demonstrating that the invention allows a more stable dispersion of the composition dispersed all over the aqueous dispersion.

**Example 6 : Ingredients of the pulverulent compositions C and D.**

The riboflavin water-dispersible pulverulent compositions C and D made in accordance with the process of the invention contain the ingredients disclosed in Table 5.

*Table 5: Ingredients of the pulverulent composition C and D.*

| Ingredient | % by total weight | Raw material reference |
|---|---|---|
| Riboflavin | 43.00 | Riboflavin (Hubei Guangji Pharmaceutical) |
| OSA | 57.00 | Sodium Octenyl Succinate starch (Cerestar) |

As a comparison, the pulverulent compositions C and D do not contain glucose syrup, unlike pulverulent compositions A and B as disclosed in the previous examples.
Pulverulent composition C was obtained after spray drying and pulverulent composition D was obtained after microgrinding followed by spray drying.

**Example 7: Detailed protocol for manufacture of water-dispersible pulverulent compositions C and D.**

The ingredients of pulverulent compositions C and D are those disclosed in example 6.
The sodium octenyl succinate (OSA) is added to water and complete dissolution is achieved. Riboflavin is then added and stirred vigorously to obtain a uniform suspension.
One part of the preparation is then grinded in a ball mixer.
The above suspensions are then spray dried. The inlet air temperature is held at about 150°C and the outlet temperature is about 100°C. The pulverulent compositions obtained disperse instantaneously in water and result in a stable suspension before uses.
Pulverulent composition C is the one after spray drying (without grinding).
Pulverulent composition D is the one after grinding and spray drying.

**Example 8 : Stability of the pulverulent compositions C and D obtained in example7**

Results of the sedimentation tests are disclosed in table 6.

*Table 6: Size of the sediment and percentage of dry content of the sediment according to the nature of the composition.*

| composition | Process | Sediment after 24H (ml) | Dry content of the sediment (%) |
|---|---|---|---|
| Riboflavin (raw material) (control) | | 1.5 | 39.4 |
| Pulverulent Composition C | Spray dried | 2.2 | 25.4 |
| Pulverulent Composition D | Grinded and Spray dried | 6.0 | 15.5 |

The results show that the sediment of the composition C is more hydrated than the raw material (25 % dry content compared to approx. 39% dry content for the raw material). The comparison of the results for pulverulent compositions C and D also demonstrates the effect of pre-grinding of the material by the important increase in moisture (only 15.5 % solids) and size of the sediment (6 ml). The composition using one saccharide allows good results for dispersion of the pulverulent composition in aqueous solution, but results are less effective than the composition A using a first saccharide and a second saccharide.

**Example 9 : Ingredients of pulverulent composition E.**

The riboflavin water-dispersible pulverulent composition E made in accordance with the invention contains the ingredients disclosed in table 7:

*Table 7: Ingredients of pulverulent composition E.*

| Ingredient | % by total weight | Raw material reference |
|---|---|---|
| Riboflavin | 43.00 | Riboflavin (Hubei Guangji Pharmaceutical) |
| Maltodextrin | 57.00 | Maltodextrin (Roquette) |

Pulverulent composition E was obtained after spray drying.

**Example 10: Detailed protocol for manufacture of water-dispersible pulverulent composition E**

The maltodextrin is added to water and complete dissolution is achieved. Riboflavin is then added and stirred vigorously to obtain a uniform suspension. The above suspension is then spray dried. The inlet air temperature is held at about 150°C and the outlet temperature is about 100°C. The pulverulent composition E obtained disperses instantaneously in water and results in a stable suspension before uses.

**Example 11 : Stability of the pulverulent composition E obtained in example 10**

The results of the sedimentation tests are given in table 8.

*Table 8: Size of the sediment and percentage of dry content of the sediment according to the nature of the composition.*

| Composition | Process | Sediment after 24H (ml) | Dry content of the sediment (%) |
|---|---|---|---|
| Riboflavin (raw material) (control) | | 1.5 | 39.4 |
| Pulverulent composition E | Spray dried | 1.3 | 32.6 |

The results show that the sediments of pulverulent composition E is more hydrated than the raw material. Nevertheless results show that composition A using two saccharides gives better results in comparison to composition E using only one saccharide.

**Example 12 : Ingredients of pulverulent composition F.**

A riboflavin water-dispersible pulverulent composition F, made in accordance with the invention, contains the ingredients disclosed in table 9.

*Table 9: Ingredients of pulverulent composition F.*

| Ingredient | % by total weight | Raw material reference |
|---|---|---|
| Riboflavin | 43.00 | Riboflavin (Hubei Guangji Pharmaceutical) |
| OSA | 14.25 | Sodium Octenyl Succinate starch (Cerestar) |
| Saccharose | 42.75 | Sugar (Beghin Say) |

As a comparison, the ingredients of pulverulent composition F are the same as those of the pulverulent composition A, except the nature of the second saccharide: glucose syrup for pulverulent composition A and saccharose for pulverulent composition F.
Pulverulent composition F was obtained after spray drying.

**Example 13: Detailed protocol for manufacture of water-dispersible pulverulent composition F**

The saccharose is added to water and complete dissolution is achieved. Riboflavin is then added and stirred vigorously to obtain a uniform suspension. The above suspension is then spray dried. The inlet air temperature is held at about 150°C and the outlet temperature is about 100°C. The pulverulent composition F obtained disperses instantaneously in water and results in a stable suspension before uses.

**Example 14 : Stability of the pulverulent composition F obtained in example 13**

The results of the sedimentation tests are given in table 10.

*Table 10: Size of the sediment and percentage of dry content of the sediment according to the nature of the composition.*

| Product | Process | Sediment after 24H (ml) | Dry content of the sediment (%) |
|---|---|---|---|
| Riboflavin (raw material) (control) | | 1.5 | 39.4 |
| Pulverulent composition F | Spray dried | 1.6 | 34.0 |

The tests show that the sedimentation is comparable between the pulverulent composition F and the control. The stability of the aqueous solution of pulverulent composition F is about the same as the one of pure riboflavin.
These results show that composition A using a second saccharides choosen among the glucose syrup types gives better results in comparison to composition F.

**Example 15 : Ingredients of pulverulent composition G.**

The riboflavin water-dispersible pulverulent composition made in accordance with the invention contains the ingredients disclosed in table 11.

*Table 11: Ingredients of pulverulent composition G.*

| Ingredient | % by total weight | Raw material reference |
|---|---|---|
| Riboflavin | 43.00 | Riboflavin (Hubei Guangji Pharmaceutical) |
| OSA | 14.25 | Sodium Octenyl Succinate starch (Cerestar) |
| Fructooligosaccharide | 42.75 | fructooligosaccharide (Orafti) |

As a comparison, the ingredients of pulverulent composition G are the same than the one of pulverulent composition A and F, except the nature of the second saccharide: glucose syrup for pulverulent composition A, saccharose for pulverulent composition F and fructooligosaccharide for pulverulent composition G.

The pulverulent composition G was obtained after spray drying.

**Example 16: Detailed protocol for manufacture of water-dispersible pulverulent composition G.**

The fructooligosaccharide is added to water and complete dissolution is achieved. Riboflavin is then added and stirred vigorously to obtain a uniform suspension. The above suspension is then spray dried. The inlet air temperature is held at about 150°C and the outlet temperature is about 100°C. The pulverulent composition G obtained disperses instantaneously in water and results in a stable suspension before uses.

**Example 17 : Stability of the pulverulent composition G obtained in example 16**

The results of sedimentation tests are given in table 12.

Table 12: Size of the sediment and percentage of dry content of the sediment according to the nature of the composition.

| Product | Process | Sediment after 24H (ml) | Dry content of the sediment (%) |
|---|---|---|---|
| Riboflavin (raw material) (control) | | 1.5 | 39.4 |
| Pulverulent composition G | Spray dried | 2.1 | 31.1 |

The results show that the sediment of the composition G is more hydrated than the raw material (31 % dry content compared to approx 39% dry content for the raw material).

**Example 18 : Ingredients of pulverulent compositions H and I**

The riboflavin water-dispersible pulverulent compositions H and I, made in accordance with the invention, contain the ingredients disclosed respectively in table 13 and 14.

Table 13: Ingredients of pulverulent composition H.

| Ingredient | % by total weight | Raw material reference |
|---|---|---|
| Riboflavin | 95.00 | Riboflavin (Hubei Guangji Pharmaceutical) |
| CMC | 5.00 | Carboxymethylcellulose (Aqualon) |

Table 14: Ingredients of pulverulent composition I.

| Ingredient | % by total weight | Raw material reference |
|---|---|---|
| Riboflavin | 70.00 | Riboflavin (Hubei Guangji Pharmaceutical) |
| CMC | 30.00 | Carboxymethylcellulose (Aqualon) |

The pulverulent compositions H and I were obtained after spray drying.

**Exemple 19: Detailed protocol for manufacture of water-dispersible pulverulent compositions H and I.**

The CMC is added to water and complete dissolution is achieved. Riboflavin is then added and stirred vigorously to obtain a uniform suspension. The above suspensions are then spray dried. The inlet air temperature is held at about 150°C and the outlet temperature is about 100°C. The pulverulent compositions H and I obtained disperse instantaneously in water and result in a stable suspension before uses.

**Example 20 : Stability of the pulverulent compositions H and I obtained in example 19**

The results of sedimentation tests are given in table 15.

*Table 15: Size of the sediment and percentage of dry content of the sediment according to the nature of the composition.*

| Product | Process | Sediment after 24H (ml) | Dry content of the sediment (%) |
|---|---|---|---|
| Riboflavin (raw material) (control) | | 1.5 | 39.4 |
| Pulverulent composition H | Spray dried | 3.1 | 32.2 |
| Pulverulent composition I | Spray dried | 5.4 | 13.3 |

The results show that the sediments of the composition H and I are more hydrated than the raw material (respectively 32 % and 13% dry content compared to approx 39% dry content for the raw material) and that hydration of the sediments is improved for pulverulent composition I compared to pulverulent composition H.

**Example 21: Ingredients of pulverulent composition J**

The riboflavin water-dispersible pulverulent composition J, made in accordance with the invention, contains the ingredients disclosed in table 16.

*Table 16: Ingredients of pulverulent composition J.*

| Ingredient | % by total weight | Raw material reference |
|---|---|---|
| Riboflavin | 43.00 | Riboflavin (Hubei Guangji Pharmaceutical) |
| Arabic Gum | 57.00 | Acacia Gum (Colloïdes Naturals International) |

As a comparison, the pulverulent composition J contains Riboflavin and only one saccharide, the Arabic Gum, possessing hydrocolloidal properties.
The pulverulent composition J was obtained after spray drying.

**Example 22: Detailed protocol for manufacture of water-dispersible pulverulent composition J obtained in example 21**

The Arabic Gum is added to water and complete dissolution is achieved. Riboflavin is then added and stirred vigorously to obtain a uniform suspension. The above suspension is then spray dried. The inlet air temperature is held at about 150°C and the outlet temperature is about 100°C. The pulverulent composition J obtained disperses instantaneously in water and results in a stable suspension before uses.

**Example 23: Stability of the pulverulent composition J obtained in example 21**

The results of sedimentation tests are given in table 17.

*Table 17: Size of the sediment and percentage of dry content of the sediment according to the nature of the composition.*

| Product | Process | Sediment after 24H (ml) | Dry content of the sediment (%) |
|---|---|---|---|
| Riboflavin (raw material) | | 1.5 | 39.4 |
| Pulverulent composition J | Spray dried | 2.0 | 33.8 |

The results show that the sediment of the composition J is more hydrated than the raw material (33 % dry content compared to approx 39% dry content for the raw material). Nevertheless results show that composition A using two saccharides gives better results in comparison to composition E using only one saccharide.

**Example 24: Ingredients of pulverulent composition K**

The riboflavin water-dispersible pulverulent composition K, made in accordance with the invention, contains the ingredients disclosed in table 18.

*Table 18: Ingredients of pulverulent composition K.*

| Ingredient | % by total weight | Raw material reference |
|---|---|---|
| Riboflavin | 95.00 | Riboflavin (Hubei Guangji Pharmaceutical) |
| Xanthan Gum | 5.00 | Xanthan Gum (Danisco) |

As a comparison, the pulverulent composition K contains Riboflavin and only one saccharide, the Xanthan Gum, possessing hydrocolloidal properties.

The pulverulent composition K was obtained after spray drying.

**Example 25 : Detailed protocol for manufacture of water-dispersible pulverulent composition J obtained in example 21**

The Xanthan Gum is added to water and complete dissolution is achieved. Riboflavin is then added and stirred vigorously to obtain a uniform suspension. The above suspension is then spray dried. The inlet air temperature is held at about 150°C and the outlet temperature is about 100°C. The pulverulent composition K obtained disperses instantaneously in water and results in a stable suspension before uses.

**Example 26 : Stability of the pulverulent composition K obtained in example 24**

The results of sedimentation tests are given in table 19.

*Table 19: Size of the sediment and percentage of dry content of the sediment according to the nature of the composition.*

| Product | Process | Sediment after 24H (ml) | Dry content of the sediment (%) |
|---|---|---|---|
| Riboflavin (raw material) | | 1.5 | 39.4 |
| Pulverulent composition K | Spray dried | 10.0 | 11.3 |

The results show that the sediment of the composition K is more hydrated than the raw material (11 % dry content compared to approx 39% dry content for the raw material).

**Example 27 : Ingredients of composition L and M**

The riboflavin water-dispersible pulverulent compositions L and M made in accordance with the invention contain the ingredients disclosed in table 20.

*Table 20: Ingredients of pulverulent composition L and M.*

| Ingredient | % by total weight | Raw material reference |
|---|---|---|
| Riboflavin | 70.0 | Riboflavin (Hubei Guangji Pharmaceutical) |
| OSA | 7.5 | Sodium Octenyl Succinate starch (Cerestar) |
| Glucose syrup | 22.5 | Glucose sirup DE 38 (Cerestar) |

As a comparison, the ingredients of pulverulent composition L and M are the same as the one of pulverulent composition A and B, but in different concentrations (respectively 70% for L and M, and 43% of riboflavin for A and B).

The pulverulent composition L was obtained after spray drying and pulverulent composition M was obtained after microgrinding followed by spray drying.

**Example 28: Detailed protocol for manufacture of water-dispersible pulverulent compositions L and M**

The OSA and glucose syrup are added to water and complete dissolution is achieved. Riboflavin is then added and stirred vigorously to obtain a uniform suspension. One part of the preparation is then grinded in a ball mixer. The above suspensions are then spray dried. The inlet air temperature is held at about 150°C and the outlet temperature is about 100°C. The pulverulent compositions obtained disperse instantaneously in water and result in a stable suspension before uses.

Pulverulent composition L is the one after spray drying (without grinding).

Pulverulent composition M is the one after grinding and spray drying.

**Example 29: Stability of the pulverulent composition L obtained in example 28**

Results of the sedimentation tests are given in table 21.

*Table 21: Size of the sediment and percentage of dry content of the sediment according to the nature of the composition.*

| Product | Process | Sediment after 24H (ml) | Dry content of the sediment (%) |
|---|---|---|---|
| Riboflavin (raw material) | | 1.5 | 39.4 |
| Pulverulent composition L | Spray dried | 2.4 | 24.9 |
| Pulverulent composition M | Grinded and Spray dried | 7.0 | 6.6 |

Results show that the sediment of the pulverulent composition L is more hydrated than the raw material (25 % dry content compared to 39% dry content for the raw material)). The effect of pre-grinding of the material is also demonstrated by the important increase in moisture (only 6.6% solids) and size of the sediment (7 ml) demonstrating that the invention allows a more stable dispersion of the composition in aqueous dispersion.

**Claims**

**1.** Pulverulent composition comprising more than 15% in weight, and less than 96% in weight, preferably from 15 to 80%, and more preferably from 20% to 70%, of at least a vitamin having a solubility lower than 500mg/L in aqueous media,

said pulverulent composition consisting in particles individually coated by at least one hydrophilic agent and having a wettability by water and dispersion shorter than 15 seconds, considering 10g of powder added to 100ml distilled water at room temperature, and

said pulverulent composition being stable on storage and having solubility properties in aqueous solutions,

said vitamin being vitamin B2 (Riboflavin),

said hydrophilic agent comprising or consisting in:

- at least a first saccharide possessing hydrocolloidal properties,

said saccharide being chosen among the group consisting of alginate, starch, modified starch, in particular octenyl succinate starch, arabic gum, and,

- at least a second saccharide, chosen among the group consisting of

- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen, starch and modified starch, fructooligosaccharide (FOS).

**2.** Pulverulent composition according to claim 1, wherein the pulverulent composition comprises from 5% to 85%, preferably 20% to 70%, and more preferably from 40% to 60% in weight of saccharide.

**3.** Pulverulent composition according to claim 1 or 2, wherein the first saccharide is octenyl succinate starch and the second saccharide is glucose syrup.

**4.** Pulverulent composition according to any of claims 1 to 3, **characterized in that** the pulverulent composition comprises granules, said granules consisting in a set of associated particles, and wherein

- said granules comprise at least a vitamin having a solubility lower than 500mg/L in aqueous media, said vitamin being vitamin B2 (Riboflavin), at least a first saccharide, possessing hydrocolloidal properties, said saccharide being chosen among the group consisting of alginate, starch, modified starch, in particular octenyl succinate starch, arabic gum, and at least a second saccharide, chosen among the group consisting of

- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen, starch and modified starch, fructooligosaccharide (FOS),

- the mean diameter of said granule is from 10 $\mu$m to 500 $\mu$m, preferably 20 to 100 $\mu$m, more preferably 30 to 50 $\mu$m,
- the span (DV05) of said granules is from 0.2 to 3.0, preferably 1.0 to 2.0, more preferably from 1.2 to 1.7,
- the macroscopic density of said pulverulent composition is from 300 to 800 g/l, preferably 400 to 700 g/l, and more preferably 400 to 600 g/l,
- the flow index determined by Flowdex method of said pulverulent composition is from 4 to 20, preferably from 5 to 10, more preferably around 8.

**5.** Pulverulent composition according to claim 4, wherein said granules comprise riboflavin, modified starch and glucose syrup.

**6.** Pulverulent composition according to claim 5, wherein said modified starch is octenyl succinate starch.

**7.** Pulverulent composition according to any of claims 4 to 6, **characterized in that** the pulverulent composition comprises granules, wherein the stability of a solution made of 1,5 g of said pulverulent composition in 8,5 g of water is such that a sediment from 1,5 ml to 10 ml, preferably 4 to 9 ml , more preferably from 6 to 8 ml is obtained after 24 hours.

**8.** Process of preparation of pulverulent compositions according to any of claims 1 to 7 comprising a step of atomisation of an aqueous phase comprising
vitamin B2 (Riboflavin), said vitamin having a solubility lower than 500mg/L in aqueous media, at least a first saccharide, possessing hydrocolloidal properties, said saccharide being chosen among the group consisting of alginate, starch, modified starch, in particular octenyl succinate starch, arabic gum, and
at least a second saccharide, chosen among the group consisting of

- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen, starch and modified starch, fructooligosaccharide (FOS).

**9.** Process of preparation of pulverulent compositions according to any of claims 1 to 7 comprising:

- a step of mixing vitamin B2 (Riboflavin) having a solubility lower than 500mg/L in an aqueous media with at least a saccharide to obtain an aqueous phase comprising a vitamin, at least one saccharide possessing hydrocolloidal properties, said saccharide being chosen among the group consisting of alginate, starch, modified starch, in particular octenyl succinate starch, arabic gum,, and at least a second saccharide, chosen among the group consisting of

- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen, starch and modified starch, fructooligosaccharide (FOS),

- a step of atomisation of said aqueous phase comprising said vitamin B2, at least said first saccharide and at least said second saccharide to obtain a mixture comprising said vitamin B2, at least said first saccharide and at least said second saccharide, said mixture being constituted by granules, and,

- possibly a step of coating of said granules to obtain a pulverulent composition.

10. Process according to any of claims 8 and 9 in which a step of microgrinding a vitamin and at least one saccharide comprised in the aqueous phase is added prior to the step of atomisation.

11. Process of preparation of pulverulent compositions according to any of claims 1 to 7 comprising:

- a step of mixing a vitamin having a solubility lower than 500mg/L in aqueous media, said vitamin being vitamin B2 (Riboflavin), with at least a first saccharide, possessing hydrocolloidal properties, said saccharide being chosen among the group consisting of alginate, starch, modified starch, in particular octenyl succinate starch, arabic gum, and at least a second saccharide, chosen among the group consisting of

- monosaccharide such as fructose, glucose, galactose,
- disaccharide such as saccharose, sucrose, lactose, maltose, trehalose and cellobiose, and,
- polysaccharide such as glucose syrup, dextrin, inulin, cellulose, glycogen, starch and modified starch, fructooligosaccharide (FOS),

to obtain an aqueous phase comprising said vitamin, at least said first saccharide and at least said second saccharide, and,
- possibly a step of microgrinding said vitamin B2 (Riboflavin), at least said first saccharide and at least said second saccharide comprised in the aqueous phase to obtain microgrinded vitamin B2, at least said first saccharide and at least said second saccharide in the aqueous phase a, and,
- a step of atomisation of the microgrinded vitamin B2, at least said first saccharide and at least said second saccharide comprised in the aqueous phase to obtain a mixture comprising said vitamin B2, at least said first saccharide and at least said second saccharide, said mixture being constituted by granules, and,
- possibly a step of coating of said granules to obtain a pulverulent composition, and,
- a step of recovery of the pulverulent composition obtained.

12. Use of a pulverulent composition according to any of claims 1 to 7 to prepare:

- liquid food products such as infantile milk, milk, acidified milk products, drinks, fruit juices and sorbet, or,
- solid food products such as bakery, noodles, bread, or,
- semi-liquid food products such as spreads, yoghourts, dips and ice creams, or,
- emulsion food products such as sauces, in particular mayonnaise and mustard, or
- powders such as dehydrated soup, dehydrated sauce, dehydrated drink preparation, powder milk, cocoa powder, instant powder drinks, meal replacement products or effervescent products, said powders being liable to be instantaneously dispersed in an aqueous solution to provide a beverage, or
- a pharmaceutical composition.

13. Liquid food product containing:

- from 0,1 mg/kg to 1500 mg/kg, preferably 1 mg/kg to 400 mg/kg, more preferably 4 mg/kg to 40 mg/kg of a pulverulent composition according to any of claims 1 to 2, and 4 to 7,
wherein:

- said liquid food product is liquid or frozen,
- said liquid food product is stable at temperatures from 6 to 60°C, preferably from 10°C to 50°C, more preferably from 20°C to 40°C during a period of 2 years,
- said pulverulent composition is homogeneously dispersed in said liquid food product,
- said vitamin does not form any deposit on the wall of a container which would contain said liquid food product,
- said liquid food product being preferably chosen among the group consisting of fruit juices and sorbet,

except liquid food product chosen from the group consisting of infantile milk, milk, acidified milk products, drinks and oils.

14. Semi-liquid food product containing:

- from 0,1 mg/kg to 1500 mg/kg, preferably 1 mg/kg to 400 mg/kg, more preferably 4 mg/kg to 40 mg/kg of a pulverulent composition according to any of claims 1 to 2, and 4 to 7,
wherein:

- said semi-liquid food product is semi-liquid or frozen,
- said semi-liquid food product is stable at temperatures from 6 to 60°C, preferably from 10°C to 50°C, more preferably from 20°C to 40°C during a period of 2 years,
- said pulverulent composition is homogeneously dispersed in said semi-liquid food product,
- said semi-liquid food product being preferably chosen among the group consisting of yoghourts, dips and ice creams,

except semi-liquid food product chosen from the group consisting of spreads and jams.

15. Emulsion food product containing:

- from 0,1 mg/kg to 1500 mg/kg, preferably 1 mg/kg to 400 mg/kg, more preferably 4 mg/kg to 40 mg/kg of a pulverulent composition according to any of claims 1 to 2, and 4 to 7,
wherein:

- said emulsion food product is liquid or frozen,
- said emulsion food product is stable at temperatures from 6 to 60°C, preferably from 10°C to 50°C, more preferably from 20°C to 40°C during a period of 2 years,
- said pulverulent composition is homogeneously dispersed in said emulsion food product,
- said emulsion food product is a sauce, in particular mayonnaise and mustard.

16. Pharmaceutical composition containing:

- an active substance contained in the pulverulent composition according to according to any of claims 1 to 2, and 4 to 7,
in association with a pharmaceutically acceptable vehicule.

**Patentansprüche**

1. Pulverförmige Zusammensetzung, umfassend mehr als 15 Gew.-%, und weniger als 96 Gew.-%, bevorzugt 15 bis 80 Gew.-%, und stärker bevorzugt 20 Gew.-% bis 70 Gew.-%, mindestens eines Vitamins mit einer Löslichkeit von weniger als 500 mg/L in wässrigen Medien,
wobei die pulverförmige Zusammensetzung aus Partikeln besteht, die einzeln mit mindestens einem hydrophilen Mittel beschichtet sind, das eine Benetzbarkeit mit Wasser und eine Dispersion von weniger als 15 Sekunden aufweist, wenn angenommen wird, dass bei Raumtemperatur 10 g Pulver zu 100 ml destilliertem Wasser gegeben werden, und
wobei die pulverförmige Zusammensetzung bei Lagerung stabil ist und Löslichkeitseigenschaften in wässrigen Lösungen aufweist,
wobei das Vitamin Vitamin B2 (Riboflavin) ist,
wobei das hydrophile Mittel Folgendes umfasst oder aus Folgendem besteht:

- mindestens einem ersten Saccharid, das hydrokolloide Eigenschaften besitzt,

wobei das Saccharid ausgewählt ist aus der Gruppe bestehend aus Alginat, Stärke, modifizierter Stärke, insbesondere Octenylsuccinatstärke, Gummi arabicum und

- mindestens einem zweiten Saccharid, ausgewählt aus der Gruppe bestehend aus

- Monosaccharid, wie etwa Fructose, Glucose, Galactose,
- Disaccharid, wie etwa Saccharose, Sucrose, Lactose, Maltose, Trehalose und Cellobiose und
- Polysaccharid, wie etwa Glucosesirup, Dextrin, Inulin, Cellulose, Glycogen, Stärke und modifizierte Stärke, Fructooligosaccharid (FOS).

2. Pulverförmige Zusammensetzung nach Anspruch 1, wobei die pulverförmige Zusammensetzung 5 Gew.-% bis 85 Gew.-%, bevorzugt 20 Gew.-% bis 70 Gew.-% und stärker bevorzugt 40 Gew.-% bis 60 Gew.-% Saccharid umfasst.

**3.** Pulverförmige Zusammensetzung nach Anspruch 1 oder. 2, wobei das erste Saccharid Octenylsuccinatstärke ist und das zweite Saccharid Glucosesirup ist.

**4.** Pulverförmige Zusammensetzung nach einem der Ansprüche 1 bis 3, **dadurch gekennzeichnet, dass** die pulverförmige Zusammensetzung Körnchen umfasst, wobei die Körnchen aus einer Reihe von assoziierten Partikeln besteht und wobei

- die Körnchen mindestens ein Vitamin mit einer Löslichkeit von weniger als 500 mg/L in wässrigen Medien, wobei das Vitamin Vitamin B2 (Riboflavin) ist, mindestens ein erstes Saccharid, das hydrokolloide Eigenschaften besitzt, wobei das Saccharid ausgewählt ist aus der Gruppe bestehend aus Alginat, Stärke, modifizierter Stärke, insbesondere Octenylsuccinatstärke, Gummi arabicum, und mindestens ein zweites Saccharid umfassen, ausgewählt aus der Gruppe bestehend aus

- Monosaccharid, wie etwa Fructose, Glucose, Galactose,
- Disaccharid, wie etwa Saccharose, Sucrose, Lactose, Maltose, Trehalose und Cellobiose und
- Polysaccharid, wie etwa Glucosesirup, Dextrin, Inulin, Cellulose, Glycogen, Stärke und modifizierte Stärke, Fructooligosaccharid (FOS),

- wobei der mittlere Durchmesser des Körnchens 10 $\mu$m bis 500 $\mu$m, bevorzugt 20 bis 100 $\mu$m, stärker bevorzugt 30 bis 50 $\mu$m beträgt,
- wobei der Span (DV05) der Körnchen 0,2 bis 3,0, bevorzugt 1,0 bis 2,0, stärker bevorzugt 1,2 bis 1,7 beträgt,
- wobei die makroskopische Dichte der pulverförmigen Zusammensetzung 300 bis 800 g/l, bevorzugt 400 bis 700 g/l und stärker bevorzugt 400 bis 600 g/l beträgt,
- wobei der mittels des Flowdex-Verfahrens bestimmte Fließfähigkeitsindex der pulverförmigen Zusammensetzung 4 bis 20, bevorzugt 5 bis 10, stärker bevorzugt etwa 8 beträgt.

**5.** Pulverförmige Zusammensetzung nach Anspruch 4, wobei die Körnchen Riboflavin, modifizierte Stärke und Glucosesirup umfassen.

**6.** Pulverförmige Zusammensetzung nach Anspruch 5, wobei es sich bei der modifizierten Stärke um Octenylsuccinatstärke handelt.

**7.** Pulverförmige Zusammensetzung nach einem der Ansprüche 4 bis 6, **dadurch gekennzeichnet, dass** die pulverförmige Zusammensetzung Körnchen umfasst, wobei die Stabilität einer Lösung, die aus 1,5 g der pulverförmigen Zusammensetzung in 8,5 g Wasser hergestellt wurde, derart ist, dass nach 24 Stunden ein Niederschlag von 1,5 ml bis 10 ml, bevorzugt 4 bis 9 ml, stärker bevorzugt 6 bis 8 ml erhalten wird.

**8.** Verfahren zur Herstellung von pulverförmigen Zusammensetzungen nach einem der Ansprüche 1 bis 7, umfassend einen Schritt zum Zerstäuben einer wässrigen Phase, umfassend
Vitamin B2 (Riboflavin), wobei das Vitamin eine Löslichkeit von weniger als 500 mg/L in wässrigen Medien aufweist, mindestens ein erstes Saccharid, das hydrokolloide Eigenschaften besitzt, wobei das Saccharid ausgewählt ist aus der Gruppe bestehend aus Alginat, Stärke, modifizierter Stärke, insbesondere Octenylsuccinatstärke, Gummi arabicum und
mindestens ein zweites Saccharid, ausgewählt aus der Gruppe bestehend aus

- Monosaccharid, wie etwa Fructose, Glucose, Galactose,
- Disaccharid, wie etwa Saccharose, Sucrose, Lactose, Maltose, Trehalose und Cellobiose und
- Polysaccharid, wie etwa Glucosesirup, Dextrin, Inulin, Cellulose, Glycogen, Stärke und modifizierte Stärke, Fructooligosaccharid (FOS).

**9.** Verfahren zur Herstellung von pulverförmigen Zusammensetzungen nach einem der Ansprüche 1 bis 7, umfassend:

- einen Schritt zum Mischen von Vitamin B2 (Riboflavin) mit einer Löslichkeit von weniger als 500 mg/L in wässrigen Medien mit mindestens einem Saccharid, um eine wässrige Phase zu erhalten, umfassend ein Vitamin und mindestens ein Saccharid, das hydrokolloide Eigenschaften besitzt, wobei das Saccharid ausgewählt ist aus der Gruppe bestehend aus Alginat, Stärke, modifizierter Stärke, insbesondere Octenylsuccinatstärke, Gummi arabicum und mindestens ein zweites Saccharid, ausgewählt aus der Gruppe bestehend aus

- Monosaccharid, wie etwa Fructose, Glucose, Galactose,
- Disaccharid, wie etwa Saccharose, Sucrose, Lactose, Maltose, Trehalose und Cellobiose und
- Polysaccharid, wie etwa Glucosesirup, Dextrin, Inulin, Cellulose, Glycogen, Stärke und modifizierte Stärke, Fructooligosaccharid (FOS),

- einen Schritt zum Zerstäuben der wässrigen Phase, umfassend ein Vitamin B2, mindestens das erste Saccharid und mindestens das zweite Saccharid, um ein Gemisch zu erhalten, umfassend das Vitamin B2, mindestens das erste Saccharide und mindestens das zweite Saccharid, wobei das Gemisch aus Körnchen besteht und
- gegebenenfalls einen Schritt zum Beschichten der Körnchen, um eine pulverförmige Zusammensetzung zu erhalten.

10. Verfahren nach einem der Ansprüche 8 und 9, worin ein Schritt zum Mikronassvermahlen eines Vitamins und mindestens eines Saccharids, die in der wässrigen Phase enthalten sind, vor dem Schritt zum Zerstäuben eingefügt wird.

11. Verfahren zur Herstellung von pulverförmigen Zusammensetzungen nach einem der Ansprüche 1 bis 7, umfassend:

- einen Schritt zum Mischen eines Vitamins mit einer Löslichkeit von weniger als 500 mg/L in wässrigen Medien, wobei das Vitamin Vitamin B2 (Riboflavin) ist, mit mindestens einem ersten Saccharid, das hydrokolloide Eigenschaften besitzt, wobei das Saccharid ausgewählt ist aus der Gruppe bestehend aus Alginat, Stärke, modifizierter Stärke, insbesondere Octenylsuccinatstärke, Gummi arabicum, und mindestens ein zweites Saccharid, ausgewählt aus der Gruppe bestehend aus

- Monosaccharid, wie etwa Fructose, Glucose, Galactose,
- Disaccharid, wie etwa Saccharose, Sucrose, Lactose, Maltose, Trehalose und Cellobiose und
- Polysaccharid, wie etwa Glucosesirup, Dextrin, Inulin, Cellulose, Glycogen, Stärke und modifizierte Stärke, Fructooligosaccharid (FOS),

um eine wässrige Phase zu erhalten, die ein Vitamin, mindestens das erste Saccharid und mindestens das zweite Saccharid umfasst, und
- gegebenenfalls einen Schritt zum Mikronassvermahlen des Vitamins B2 (Riboflavin), mindestens des ersten Saccharids und mindestens des zweiten Saccharids, die in der wässrigen Phase enthalten sind, um mikronassvermahlenes Vitamin B2, mindestens das erste Saccharid und mindestens das zweite Saccharids in der wässrigen Phase zu erhalten, und
- einen Schritt zum Zerstäuben des mikronassvermahlenen Vitamins B2, mindestens des ersten Saccharids und mindestens des zweiten Saccharids, die in der wässrigen Phase enthalten sind, um ein Gemisch zu erhalten, das das Vitamin B2, mindestens das erste Saccharid und mindestens das zweite Saccharid umfasst, wobei das Gemisch aus Körnchen besteht und
- gegebenenfalls einen Schritt zum Beschichten der Körnchen, um eine pulverförmige Zusammensetzung zu erhalten und
- einen Schritt zum Gewinnen der erhaltenen pulverförmigen Zusammensetzung.

12. Verwendung einer pulverförmigen Zusammensetzung nach einem der Ansprüche 1 bis 7, um Folgendes herzustellen:

- flüssige Nahrungsmittelprodukte, wie etwa Säuglingsmilch, Milch, Sauermilchprodukte, Getränke, Fruchtsäfte und Sorbet oder
- feste Nahrungsmittelprodukte, wie etwa Backwaren, Nudeln, Brot oder
- halbflüssige Nahrungsmittelprodukte, wie etwa Brotaufstriche, Joghurts, Dips und Eiscremes oder
- emulgierte Nahrungsmittelprodukte, wie etwa Saucen, insbesondere Mayonnaise und Senf oder
- Pulver, wie etwa dehydrierte Suppe, dehydrierte Sauce, dehydrierte Getränkezubereitung, Milchpulver, Kakaopulver, Instant-Pulver-Getränke, Mahlzeitenersatzprodukte oder Brauseprodukte, wobei die Pulver in einer wässrigen Lösung sofort dispergierbar sein müssen, um ein Getränk bereitzustellen oder
- eine pharmazeutische Zusammensetzung.

13. Flüssiges Nahrungsmittelprodukt, enthaltend:

- 0,1 mg/kg bis 1.500 mg/kg, bevorzugt 1 mg/kg bis 400 mg/kg, stärker bevorzugt 4 mg/kg bis 40 mg/kg einer pulverförmigen Zusammensetzung nach einem der Ansprüche 1 bis 2 und 4 bis 7,
wobei:
- das flüssige Nahrungsmittelprodukt flüssig oder gefroren ist,
- wobei das flüssige Nahrungsmittelprodukt bei Temperaturen von 6 bis 60 °C, bevorzugt 10 °C bis 50 °C, stärker bevorzugt 20 °C bis 40 °C während eines Zeitraums von 2 Jahren stabil ist,
- wobei die pulverförmige Zusammensetzung in dem flüssigen Nahrungsmittelprodukt homogen dispergiert ist,
- wobei das Vitamin an der Wand eines Behälters, der das flüssige Nahrungsmittelprodukt enthält, keine Ablagerung bildet,
- wobei das flüssige Nahrungsmittelprodukt bevorzugt ausgewählt ist aus der Gruppe bestehend aus Fruchtsäften und Sorbet,

abgesehen von dem flüssigen Nahrungsmittelprodukt, ausgewählt aus der Gruppe bestehend aus Säuglingsmilch, Milch, Sauermilchprodukten, Getränken und Ölen.

14. Halbflüssiges Nahrungsmittelprodukt, enthaltend:

- 0,1 mg/kg bis 1.500 mg/kg, bevorzugt 1 mg/kg bis 400 mg/kg, stärker bevorzugt 4 mg/kg bis 40 mg/kg einer pulverförmigen Zusammensetzung nach einem der Ansprüche 1 bis 2 und 4 bis 7,
wobei:
- das halbflüssige Nahrungsmittelprodukt halbflüssig oder gefroren ist,
- wobei das halbflüssige Nahrungsmittelprodukt bei Temperaturen von 6 bis 60 °C, bevorzugt 10 °C bis 50 °C, stärker bevorzugt 20 °C bis 40 °C während eines Zeitraums von 2 Jahren stabil ist,
- wobei die pulverförmige Zusammensetzung in dem halbflüssigen Nahrungsmittelprodukt homogen dispergiert ist,
- wobei das halbflüssige Nahrungsmittelprodukt bevorzugt ausgewählt ist aus der Gruppe bestehend aus Joghurts, Dips und Eiscremes,

abgesehen von dem halbflüssigen Nahrungsmittelprodukt, ausgewählt aus der Gruppe, bestehend aus Aufstrichen und Konfitüren.

15. Emulgiertes Nahrungsmittelprodukt, enthaltend:

- 0,1 mg/kg bis 1.500 mg/kg, bevorzugt 1 mg/kg bis 400 mg/kg, stärker bevorzugt 4 mg/kg bis 40 mg/kg einer pulverförmigen Zusammensetzung nach einem der Ansprüche 1 bis 2 und 4 bis 7,
wobei:
- das emulgierte Nahrungsmittelprodukt flüssig oder gefroren ist,
- wobei das emulgiert Nahrungsmittelprodukt bei Temperaturen von 6 bis 60 °C, bevorzugt 10 °C bis 50 °C, stärker bevorzugt 20 °C bis 40 °C während eines Zeitraums von 2 Jahren stabil ist,
- wobei die pulverförmige Zusammensetzung in dem emulgierten Nahrungsmittelprodukt homogen dispergiert ist,
- wobei das emulgierte Nahrungsmittelprodukt eine Sauce, insbesondere Mayonnaise und Senf ist.

16. Pharmazeutische Zusammensetzung, enthaltend:

- einen Wirkstoff, der in der pulverförmigen Zusammensetzung nach einem der Ansprüche 1 bis 2 und 4 bis 7 enthalten ist,

in Verbindung mit einem pharmazeutisch verträglichen Vehikel.

**Revendications**

1. Composition pulvérulente comprenant plus de 15% en poids, et moins de 96% en poids, de préférence de 15 à 80%, et plus préférablement de 20% à 70%, d'au moins une vitamine ayant une solubilité inférieure à 500 mg/l en milieu aqueux,
ladite composition pulvérulente consistant en particules enrobées individuellement par au moins un agent hydrophile et ayant une mouillabilité par l'eau et une dispersion inférieure à 15 secondes, considérant 10 g de poudre ajoutés à 100 ml d'eau distillée à température ambiante, et ladite composition pulvérulente étant stable au stockage et ayant des propriétés de solubilité dans les solutions aqueuses,
ladite vitamine étant la vitamine B2 (riboflavine),
ledit agent hydrophile comprenant ou consistant en:

- au moins un premier saccharide possédant des propriétés hydrocolloïdes,

ledit saccharide étant choisi parmi le groupe constitué de l'alginate, l'amidon, l'amidon modifié, en particulier l'octénylsuccinate d'amidon, la gomme arabique, et,

- au moins un deuxième saccharide, choisi parmi le groupe constitué :
- des monosaccharides tels que le fructose, le glucose, le galactose,
- des disaccharides tels que le saccharose, le sucrose, le lactose, le maltose, le tréhalose et le cellobiose, et,
- des polysaccharides tels que le sirop de glucose, la dextrine, l'inuline, la cellulose, le glycogène, l'amidon et l'amidon modifié, le fructooligosaccharide (FOS).

2. Composition pulvérulente selon la revendication 1, dans laquelle la composition pulvérulente comprend de 5% à 85%, de préférence de 20% à 70%, et plus préférablement de 40% à 60% en poids de saccharide.

3. Composition pulvérulente selon la revendication 1 ou 2, dans laquelle le premier saccharide est l'octénylsuccinate d'amidon et le deuxième saccharide est le sirop de glucose.

4. Composition pulvérulente selon l'une quelconque des revendications 1 à 3, **caractérisée en ce que** la composition pulvérulente comprend des granulés, lesdits granulés consistant en un ensemble de particules associées, et dans laquelle

- lesdits granulés comprennent au moins une vitamine ayant une solubilité inférieure à 500 mg/l en milieu aqueux, ladite vitamine étant la vitamine B2 (riboflavine), au moins un premier saccharide, possédant des propriétés hydrocolloïdes, ledit saccharide étant choisi parmi le groupe constitué de l'alginate, l'amidon, l'amidon modifié, en particulier l'octénylsuccinate d'amidon, la gomme arabique, et au moins un deuxième saccharide, choisi parmi le groupe constitué

- des monosaccharides tels que le fructose, le glucose, le galactose,
- des disaccharides tels que le saccharose, le sucrose, le lactose, le maltose, le tréhalose et le cellobiose, et,
- des polysaccharides tels que le sirop de glucose, la dextrine, inuline, la cellulose, le glycogène, l'amidon et l'amidon modifié, le fructooligosaccharide (FOS),

- le diamètre moyen desdites granulés est de 10 $\mu$m à 500 $\mu$m, de préférence de 20 à 100 $\mu$m, plus préférablement de 30 à 50 $\mu$m,
- l'intervalle (DV05) desdits granulés est de 0,2 à 3,0, de préférence de 1,0 à 2,0, plus préférablement de 1,2 à 1,7,
- la densité macroscopique de ladite composition pulvérulente est de 300 à 800 g/l, de préférence de 400 à 700 g/l, et plus préférablement de 400 à 600 g/l,
- L'indice de fluidité déterminée selon la méthode Flowdex de ladite composition pulvérulente est de 4 à 20, de préférence de 5 à 10, plus préférablement d'environ 8.

5. Composition pulvérulente selon la revendication 4, dans laquelle lesdits granulés comprennent de la riboflavine, de l'amidon modifié et de sirop de glucose.

6. Composition pulvérulente selon la revendication 5, dans laquelle ledit amidon modifié est l'octénylsuccinate d'amidon.

7. Composition pulvérulente selon l'une quelconque des revendications 4 à 6, **caractérisée en ce que** la composition pulvérulente comprend des granulés, dans laquelle la stabilité d'une solution faite de 1,5 g de ladite composition pulvérulente dans 8,5 g d'eau est telle qu'un sédiment de 1,5 ml à 10 ml, de préférence de 4 à 9 ml, plus préférablement de 6 à 8 ml, est obtenu après 24 heures.

8. Procédé de préparation de compositions pulvérulentes selon l'une quelconque des revendications 1 à 7, comprenant une étape d'atomisation d'une phase aqueuse comprenant de la vitamine B2 (riboflavine), ladite vitamine ayant une solubilité inférieure à 500 mg/l en milieu aqueux, au moins un premier saccharide, possédant des propriétés hydrocolloïdes, ledit saccharide étant choisi parmi le groupe constitué de l'alginate, l'amidon, l'amidon modifié, en particulier l'octénylsuccinate d'amidon, la gomme arabique, et
au moins un deuxième saccharide, choisi parmi le groupe constitué

- des monosaccharides tels que le fructose, le glucose, le galactose,
- des disaccharides tels que le saccharose, le sucrose, le lactose, le maltose, le tréhalose et le cellobiose, et,
- des polysaccharides tels que le sirop de glucose, la dextrine, inuline, la cellulose, le glycogène, l'amidon et l'amidon modifié, le fructooligosaccharide (FOS).

9. Procédé de préparation de compositions pulvérulentes selon l'une quelconque des revendications 1 à 7, comprenant:

- une étape de mélange de la vitamine B2 (riboflavine) ayant une solubilité inférieure à 500 mg/l dans un milieu aqueux avec au moins un saccharide pour obtenir une phase aqueuse comprenant une vitamine, au moins un saccharide possédant des propriétés hydrocolloïdes, ledit saccharide étant choisi parmi le groupe constitué de l'alginate, l'amidon, l'amidon modifié, en particulier l'octénylsuccinate d"amidon, la gomme arabique, et au moins un deuxième saccharide, choisi parmi le groupe constitué

- des monosaccharides tels que le fructose, le glucose, le galactose,
- des disaccharides tels que le saccharose, le sucrose le lactose, le maltose, le tréhalose et le cellobiose, et,
- des polysaccharides tels que le sirop de glucose, la dextrine, l'inuline, la cellulose, le glycogène, l'amidon et l'amidon modifié, le fructooligosaccharide (FOS),

- une étape d'atomisation de ladite phase aqueuse comprenant ladite vitamine B2, au moins ledit premier saccharide et au moins ledit deuxième saccharide pour obtenir un mélange comprenant ladite vitamine B2, au moins ledit premier saccharide et au moins ledit deuxième saccharide, ledit mélange étant constitué de granulés, et,
- éventuellement une étape d'enrobage desdits granulés pour obtenir une composition pulvérulente.

10. Procédé selon l'une quelconque des revendications 8 et 9, dans lequel une étape de microbroyage d'une vitamine et d'au moins un saccharide compris dans la phase aqueuse est ajoutée avant l'étape d'atomisation.

11. Procédé de préparation de compositions pulvérulentes selon l'une quelconque des revendications 1 à 7, comprenant:

- une étape de mélange d'une vitamine ayant une solubilité inférieure à 500 mg/l en milieu aqueux, ladite vitamine étant la vitamine B2 (riboflavine), avec au moins un premier saccharide, possédant des propriétés hydrocolloïdes, ledit saccharide étant choisi parmi le groupe constitué de l'alginate, l'amidon, l'amidon modifié, en particulier l'octénylsuccinate d'amidon, la gomme arabique, et au moins un deuxième saccharide, choisis parmi le groupe constitué

- des monosaccharides tels que le fructose, le glucose, le galactose,
- des disaccharides tels que le saccharose, le sucrose, le lactose, le maltose, le tréhalose et le cellobiose, et,
- des polysaccharides tels que le sirop de glucose, la dextrine, l'inuline, la cellulose, le glycogène, l'amidon et l'amidon modifié, le fructooligosaccharide (FOS),

pour obtenir une phase aqueuse comprenant ladite vitamine, au moins ledit premier saccharide et au moins ledit deuxième saccharide, et,
- éventuellement une étape de microbroyage de ladite vitamine B2 (riboflavine), d'au moins ledit premier saccharide et d'au moins ledit deuxième composé saccharide dans la phase aqueuse pour obtenir de la vitamine B2 microbroyée, au moins ledit premier saccharide et au moins ledit deuxième saccharide dans la phase aqueuse, et,
- une étape d'atomisation de la vitamine B2 microbroyée, d'au moins ledit premier saccharide et d'au moins ledit deuxième composé saccharide dans la phase aqueuse pour obtenir un mélange comprenant ladite vitamine B2, au moins ledit premier saccharide et au moins ledit deuxième saccharide, ledit mélange étant constituée par des granulés, et,
- éventuellement une étape d'enrobage desdits granulés pour obtenir une composition pulvérulente, et,
- une étape de récupération de la composition pulvérulente obtenue.

**12.** Utilisation d'une composition pulvérulente selon l'une quelconque des revendications 1 à 7 pour préparer:

- des produits alimentaires liquides tels que le lait infantile, le lait, les produits laitiers acidifiés, les boissons, les jus de fruits et sorbets, ou,
- des produits alimentaires solides tels que les produits de boulangerie, les pâtes, le pain, ou,
- des produits alimentaires semi-liquides tels que les pâtes à tartiner, les yaourts, les trempettes et les crèmes glacées, ou,
- des produits alimentaires en émulsions tels que les sauces, en particulier la mayonnaise et la moutarde, ou
- des poudres telles que la soupe déshydratée, la sauce déshydraté, les préparations déshydratées pour boissons, le lait en poudre, la poudre de cacao, les boissons en poudre instantanée, les produits substituts de repas ou les produits effervescents, lesdites poudres étant susceptible d'être instantanément dispersées dans une solution aqueuse pour fournir une boisson, ou
- une composition pharmaceutique.

**13.** Produit alimentaire liquide contenant:

- de 0,1 mg/kg à 1500 mg/kg, de préférence de 1 mg/kg à 400 mg/kg, plus préférablement de 4 mg/kg à 40 mg/kg d'une composition pulvérulente selon l'une quelconque des revendications 1 à 2, et 4 à 7, dans lequel:

    - ledit produit alimentaire liquide est liquide ou congelé,
    - ledit produit alimentaire liquide est stable à des températures allant de 6 à 60°C, de préférence de 10°C à 50°C, plus préférablement de 20°C à 40°C pendant une durée de 2 ans,
    - ladite composition pulvérulente est dispersée de façon homogène dans ledit produit alimentaire liquide,
    - ladite vitamine ne forme pas de dépôt sur les parois d'un récipient qui contiendrait ledit produit alimentaire liquide,
    - ledit produit alimentaire liquide est de préférence choisi parmi le groupe constitué des jus de fruits et sorbets,

à l'exception des produits alimentaires liquides choisis dans le groupe constitué du lait infantile, du lait, des produits laitiers acidifiés, des boissons et des huiles.

**14.** Produit alimentaire semi-liquide contenant:

- de 0,1 mg/kg à 1500 mg/kg, de préférence de 1 mg/kg à 400 mg/kg, plus préférablement de 4 mg/kg à 40 mg/kg d'une composition pulvérulente selon l'une quelconque des revendications 1 à 2, et 4 à 7, dans lequel:

    - ledit produit alimentaire semi-liquide est semi-liquide ou congelé,
    - ledit produit alimentaire semi-liquide est stable à des températures allant de 6 à 60°C, de préférence de 10°C à 50°C, plus préférablement de 20°C à 40°C pendant une durée de 2 ans,
    - ladite composition pulvérulente est dispersée de façon homogène dans ledit produit alimentaire semi-liquide,
    - ledit produit alimentaire semi-liquide est de préférence choisi parmi le groupe constitué des yoghourts, des trempettes et des crèmes glacées,

à l'exception des produits alimentaires semi-liquides choisis dans le groupe constitué des pâtes à tartiner et des confitures.

**15.** Produit alimentaire en émulsion contenant:

- de 0,1 mg/kg à 1500 mg/kg, de préférence de 1 mg/kg à 400 mg/kg, plus préférablement de 4 mg/kg à 40 mg/kg d'une composition pulvérulente selon l'une quelconque des revendications 1 à 2, et 4 à 7, dans lequel:

    - ledit produit alimentaire en émulsion est liquide ou congelé,
    - ledit produit alimentaire en émulsion est stable à des températures allant de 6 à 60°C, de préférence de 10°C à 50°C, plus préférablement de 20°C à 40°C pendant une durée de 2 ans,
    - ladite composition pulvérulente est dispersée de façon homogène dans ledit produit alimentaire en émulsion,
- ledit produit alimentaire en émulsion est une sauce, en particulier la mayonnaise et la moutarde.

**16.** Composition pharmaceutique contenant:

- une substance active contenue dans la composition pulvérulente selon l'une selon l'une quelconque des revendications 1 à 2, et 4 à 7,
en association avec un véhicule pharmaceutiquement acceptable.

HV | Mag | Spot | Pressure | WD | 50.0μm
20.0 kV | 800x | 4.0 | --- | 10.7 mm | matiere premiere B2

**Figure 1**

HV | Mag | Spot | Pressure | WD | 300.0μm
20.0 kV | 200x | 4.0 | --- | 10.7 mm | B2 instant

**Figure 2**

**% back scattering**

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 1211662

**Non-patent literature cited in the description**

- **GIOIA A.** Intrinsic flowability: a new technology for powder flowability classification. *Pharmaceut. Technol.*, 1980, 1-4